# EUROPEAN PATENT SPECIFICATION

(11) **EP 3 204 002 B1**
(45) Date of publication and mention of the grant of the patent: **19.05.2021**
(21) Application number: 15787905.7
(22) Date of filing: 06.10.2015
(51) Int. Cl.: A61K 31/282, A61K 31/337, A61K 31/395, A61K 31/404, A61K 31/4178, A61K 31/4725, A61K 31/506, A61K 31/513, A61K 31/65, A61P 35/00

(54) **PHARMACEUTICAL COMBINATION FOR THE TREATMENT OF TUMORS**
PHARMAZEUTISCHE KOMBINATIONEN ZUR BEHANDLUNG VON TUMOREN
COMBINAISON PHARMACEUTIQUE POUR LE TRAITEMENT DE TUMEURS

(30) Priority: 06.10.2014 IT MI20141746
(43) Date of publication of application: 16.08.2017
(73) Proprietor: International Society for Drug Development S.r.l., 20135 Milano (IT)
(72) Inventor: RAPPOSELLI, Simona, 55012 Lucca (IT); MARTINI, Claudia, 56126 Pisa (IT); CALDERONE, Vincenzo, 54100 Massa (IT); PURICELLI, Guido, 20135 Milano (IT)
(74) Representative: Cattaneo, Elisabetta
(86) International application number: PCT/EP2015/073017
(87) International publication number: WO 2016/055454

(56) References cited:
- WO-A2-2005/040116
- FR-A1- 2 821 358
- GIULIA NESI ET AL: "Synthesis of Novel 3,5-Disubstituted-2-oxindole Derivatives As Antitumor Agents against Human Nonsmall Cell Lung Cancer", ACS MEDICINAL CHEMISTRY LETTERS, vol. 4, no. 12, 12 December 2013 (2013-12-12), pages 1137-1141, XP055190819, ISSN: 1948-5875, DOI: 10.1021/ml400162g cited in the application
- JEFFREY C. HENISE ET AL: "Irreversible Nek2 Kinase Inhibitors with Cellular Activity", JOURNAL OF MEDICINAL CHEMISTRY, vol. 54, no. 12, 23 June 2011 (2011-06-23) , pages 4133-4146, XP055190816, ISSN: 0022-2623, DOI: 10.1021/jm200222m
- HIREN PATEL ET AL: "Synthesis of hybrid anticancer agents based on kinase and histone deacetylase inhibitors", MEDCHEMCOMM, vol. 5, no. 12, 18 June 2014 (2014-06-18), pages 1829-1833, XP055190821, ISSN: 2040-2503, DOI: 10.1039/C4MD00211C
- MOHAMED DIWAN M. ABDULHAMEED ET AL: "Combined 3D-QSAR Modeling and Molecular Docking Study on Indolinone Derivatives as Inhibitors of 3-Phosphoinositide-Dependent Protein Kinase-1", JOURNAL OF CHEMICAL INFORMATION AND MODELING, vol. 48, no. 9, 22 September 2008 (2008-09-22), pages 1760-1772, XP055190840, ISSN: 1549-9596, DOI: 10.1021/ci800147v
- ISLAM ET AL: "Indolinone based phosphoinositide-dependent kinase-1 (PDK1) inhibitors. Part 1: Design, synthesis and biological activity", BIOORGANIC & MEDICINAL CHEMISTRY LETTERS, PERGAMON, AMSTERDAM, NL, vol. 17, no. 14, 19 June 2007 (2007-06-19) , pages 3814-3818, XP022119960, ISSN: 0960-894X, DOI: 10.1016/J.BMCL.2007.04.071
- M. H. COHEN: "Food and Drug Administration Drug Approval Summary: Temozolomide Plus Radiation Therapy for the Treatment of Newly Diagnosed Glioblastoma Multiforme", CLINICAL CANCER RESEARCH, vol. 11, no. 19, 1 October 2005 (2005-10-01), pages 6767-6771, XP055049393, ISSN: 1078-0432, DOI: 10.1158/1078-0432.CCR-05-0722

## Description

### FIELD OF THE INVENTION

The present invention concerns a pharmaceutical combination of at least one 2-oxo-indole derivative of formula (I) and at least one antitumor drug. The combination of the invention is effective in the treatment of tumors, preferably in the treatment of glioblastoma, breast tumor and pancreatic tumor.

### STATE OF THE ART

Despite the ongoing development of new therapeutic treatments, such as surgery, radiotherapy and chemotherapy, some tumors, including glioblastoma multiforme (abbreviated to GBM), continue to be aggressive and lethal tumors.

In view of the disappointing results achieved by conventional therapies, the search for possible alternative therapies able to significantly improve the outcome of patients in these aggressive tumors is an evident need felt by cancer researchers.

The growing understanding of the complex biological networks (molecular pathways) relevant for the development and progression of the tumor led to the identification of several pharmacological targets and stimulated research to develop new "targeted therapies" aimed at more effective treatment of unresponsive tumors with respect to conventional therapies or at preventing the onset of recurrences.

The process of carcinogenesis is the result of an imbalance between the physiological phenomena of cell division and growth and the normal process of programmed death (apoptosis). In the context of this delicate balance, proteins and signal transduction pathways which regulate growth, cell differentiation and development often undergo genetic alterations which induce oncogenic modifications. The pathway of PI3K/Akt/mTOR is one of the most important intracellular signaling pathways involved in the mechanisms of cell growth and survival. This pathway is based on the cascade sequence of particular phosphorylation reactions, assured by various proteins with protein-kinase action, the main ones being PI3K, Akt and mTOR. This signaling cascade is constitutively expressed and hyperactive in many types of cancer, due to loss of the functionality of the PTEN factor (tumor suppressor), the amplification or mutation of PI3K, the amplification or mutation of Akt, the activation of receptors of the growth factors or exposure to carcinogenic agents. Once activated, it can be propagated through phosphorylation (activation) of a wide range of downstream effectors, among which the role of mTOR, a protein translocation regulator, is emerging. In particular, it has been observed that this pathway is hyperactive in many tumor forms, such as glioblastoma, multiple myeloma, tumor of the lungs, multiple myeloma, lung cancer, cancer of the head and neck, breast cancer, stomach cancer, acute myeloid leukemia, endometrial cancer, melanoma, kidney cancer, ovarian cancer, prostate cancer and cancer of the colon. Furthermore, the pathway' hyperactivity characterizes numerous tumor forms with fatal prognosis: the phosphorylation of Akt at the level of serine 473 (S473), and therefore the activation of this pathway, has been associated with an unfavorable prognosis in some tumor forms such as NSCLC tumors (non-small cell lung cancer), skin cancer (Dai DL, Martinka M, Li G.J Clin Oncol. 2005 Mar 1;23(7):1473-82), pancreas cancer (Schlieman et al., Incidence, mechanism and prognostic value of activated AKT in pancreas cancer Br J Cancer. 2003 Dec 1;89(11):2110-5; Yamamoto S et al., Prognostic significance of activated Akt expression in pancreatic ductal adenocarcinoma. Clin Cancer Res. 2004 Apr 15;10(8):2846-50), liver cancer (Nakanishi K, et al. Akt phosphorylation is a risk factor for early disease recurrence and poor prognosis in hepatocellular carcinoma. Cancer. 2005 Jan 15;103(2):307-12), prostate cancer (Kreisberg JI et al. Phosphorylation of Akt (Ser473) is an excellent predictor of poor clinical outcome in prostate cancer. Cancer Res. 2004;64(15):5232-6), breast cancer (Perez-Tenorio and Stal, Activation of AKT/PKB in breast cancer predicts a worse outcome among endocrine treated patients. Br J Cancer. 2002;86(4):540-5), endometrial cancer (Terakawa et al., Loss of PTEN expression followed by Akt phosphorylation is a poor prognostic factor for patients with endometrial cancer. Endocr Relat Cancer. 2003;10(2):203-82003), stomach cancer (Nam et al., Akt/PKB activation in gastric carcinomas correlates with clinicopathologic variables and prognosis. APMIS. 2003;111(12):1105-13.), brain cancer (Ermoian et al., 2002), and blood cancer. The hyperactivation of the PI3K/Akt/mTOR pathway is furthermore one of the factors responsible for the onset of resistance to many chemotherapy treatments.

Given the importance of the PI3K/Akt/mTOR axis in the etiopathogenesis of numerous cancer types, logically speaking, in the latest antitumor therapeutic approaches, each of these proteins should be considered an optimal molecular target for the design and synthesis of new drugs. Pharmacological treatments of this type are currently considered more effective antineoplastic therapies than the conventional therapy [Cheng J.Q., Lindsley CW, Cheng GZ, Yang H, Nicosia SV. (2005). The Akt/PKB pathway: molecular target for cancer drug discovery. Oncogene;24(50):7482-92], especially in the case of treatment of neoplasias which do not respond to chemotherapy and/or radiotherapy.

Therefore, in line with the need to provide new therapeutic/pharmacological approaches which are alternative and/or complementary to traditional chemotherapy, and on the basis of the latest knowledge on the role of the PI3K/Akt/mTOR pathway in tumor pathologies, new molecules have been synthesized able to interfere with the activity of these cell pathways, in particular with the PI3K-Akt-mTOR pathway.

For said purpose 2-oxo-indole derivative compounds were prepared. These compounds showed to be able to interfere with the activity of this pathway, thus constituting new hypotheses of molecules with antitumor activity vis-à-vis lung tumor (Nesi et al, ACS Medicinal Chemistry Letters, "Synthesis of Novel 3,5-disubstituted-2-oxindole derivative as antitumor agents against Non-Small Cell Lung cancer", 2013).

Specifically, the 2-oxo-indole derivative compounds showed a powerful antiproliferative activity associated with inhibition of the phosphorylation of Akt and with the block of the cell cycle at the G1/S phase in human NSCLC (Non-Small Cell Lung Cancer) cells.

In line with the pressing need to provide new therapeutic/pharmacological approaches which are alternative and/or complementary to traditional chemotherapy, the object of the present invention is to provide alternative therapeutic approaches vis-à-vis aggressive tumor forms such as glioblastoma.

### SUMMARY OF THE INVENTION

With the intention of identifying new drugs, the inventors of the present invention have surprisingly identified a combination of traditional drugs with molecules able to act against some effectors involved in transduction of the cell signal, which has proved to be more effective than the mono-therapy treatment.

The object of the invention has therefore been achieved by means of a combination comprising at least one 2-oxo-indole derivative of formula (I) 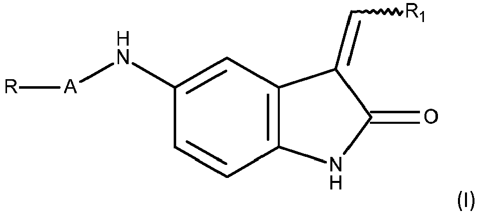 wherein
R₁ is selected from the group consisting of thienyl, imidazolyl and pyridyl, optionally substituted by (C₁-C₃) alkyl,
A is a -CH₂CO-o-SO₂- group;
R is selected from the group consisting of 6,7-dimethoxy-1,2,3,4-tetrahydroisoquinolinyl, 3,4-dimethoxy-benzylamino, (C₁-C₃) alkyl, benzyl or a pharmaceutically acceptable salt thereof and temozolomide.

The idea of the inventors of the present invention was therefore to test a combination comprising one or more molecules able to interfere with the effectors involved in the PI3K-Akt-mTOR pathway in order to improve the activity of the antitumor agent, overcoming the resistance which limits the effectiveness thereof. Said combination advantageously had a synergic effect with respect to the activity of the single elements constituting the combination, overcoming the effectiveness limits of the mono-therapy treatment.

The invention will now be described in detail and subsequently exemplified in the experimental part.

### DESCRIPTION OF THE FIGURES

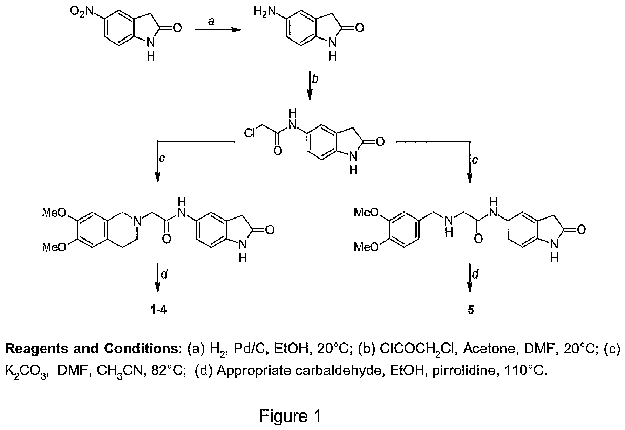

Figure 1 shows the scheme 1 for preparation of the compounds 1-5.

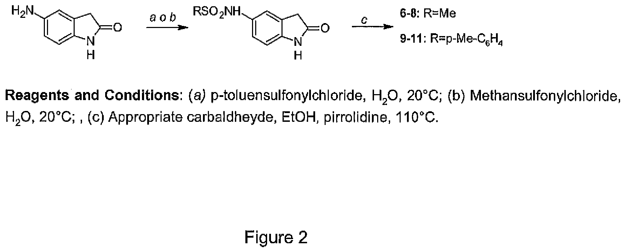

Figure 2 shows the scheme 2 for preparation of the compounds 6-11.

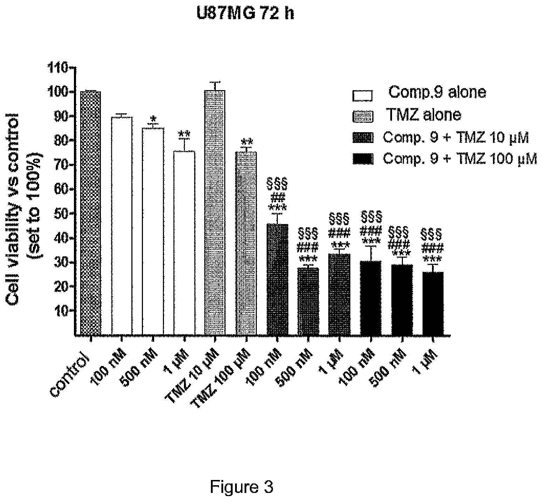

Figure 3 shows the results of the cell viability in the MTS assay of the combination of compound 9 of the invention and of temozolomide. The U87MG cells were treated with the compound 1 (100 nM, 500 nM, 1 µM) and/or with TMZ (10 or 100 µM) for 72h. At the end of the treatments, the cell viability was evaluated using the MTS assay. The values are expressed as a % with respect to the control, and represent the mean value ± SEM. The statistical analysis was conducted using the ANOVA/Bonferroni one-way test. *P<0.05, ** P<0.01, *** P<0.001 vs. control cells; ## P<0.01, ### P<0.001 vs. compound 9 only; §§§ P<0.001 vs. TMZ only.

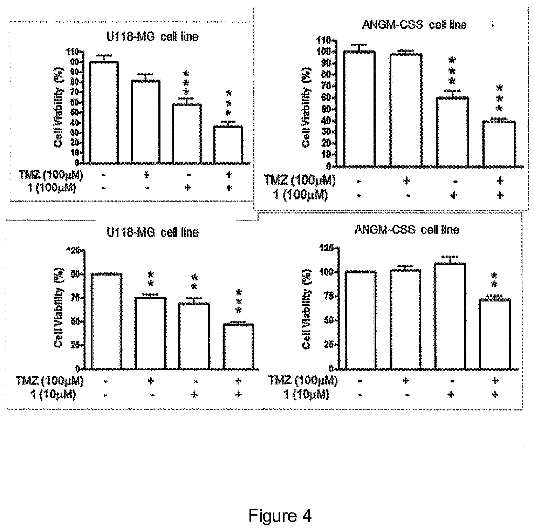

Figure 4 shows the results of the cell viability in the WST1 (Water Soluble Tetrazolium Salt 1) assay of the combination of compound 1 of the invention and of temozolomide. The graphs show the inhibitory effect produced by temozolomide (TMZ, 100µM), by compound 1 (at the concentrations 10 and 100 µM), by the association of the two compounds (TMZ 100µM + compound 1 100 µM) or by the association of the two compounds (TMZ 100µM + compound 1 10 µM), on the human cell lines U118-MG and ANGM-CSS of glioblastoma. The cell viability was evaluated using the WST1 (Water Soluble Tetrazolium Salt 1) assay. The values are expressed as a % with respect to the control, and represent the mean value ± SEM. The statistical analysis was conducted using the ANOVA/Bonferroni one-way test. ** P<0.01, *** P<0.001 vs. control cells.

### DETAILED DISCLOSURE OF THE INVENTION

The invention therefore concerns a combination comprising at least one 2-oxo-indole derivative of formula (I) 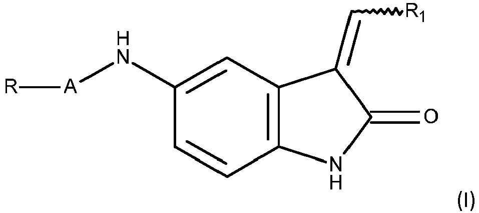 wherein
R₁ is selected from the group consisting of thienyl, imidazolyl and pyridyl, optionally substituted by (C₁-C₃) alkyl,
A is a -CH₂CO-o-SO₂- group;
R is selected from the group consisting of 6,7-dimethoxy-1,2,3,4-tetrahydroisoquinolinyl, 3,4-dimethoxy-benzylamino, (C₁-C₃)alkyl, benzyl or a pharmaceutically acceptable salt thereof
and temozolomide.

In the oxo-indole derivative of Formula (I), R₁ is selected from the group consisting of thienyl, imidazolyl and pyridyl, preferably it is thienyl or imidazolyl. A can be a methyl carbonyl or sulfonyl group. When A is -SO₂-, R₁ is preferably imidazolyl.

R is preferably selected from the group consisting of 6,7-dimethoxy-1,2,3,4-tetrahydroisoquinolinyl, 3,4-dimethoxy-benzylamino, methyl and benzyl, more preferably it is 6,7-dimethoxy-1,2,3,4-tetrahydroisoquinolinyl or benzyl.

At least one 2-oxoindole derivative of the invention is preferably a compound selected from the group consisting of

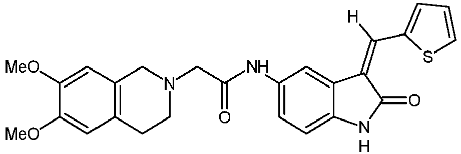

### N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide

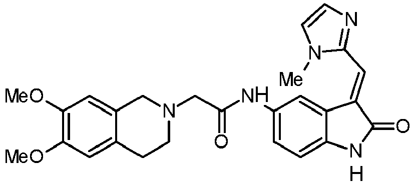

### N-[(3E)-(2-oxo-3-((1-methyl-1H-imidazol-2yl)methylene)-2,3-dihydro-1H-indol-5-yl]-2-(6,7-dimethoxy-3,4-dihydroisoquinol-2-(1H)-yl)-acetamide

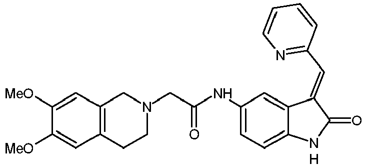

### N-[(3E)-(2-oxo-3-((pyridine-2-yl)methylene)-2,3-dihydro-1H-indol-5-yl]-2-(6,7-dimethoxy-3,4-dihydroisoquinolin-2-(1H)-yl)- indolin-5-yl)acetamide

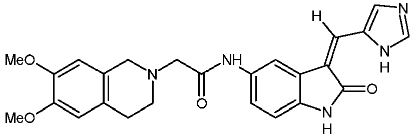

### N-[(3Z)-(2-oxo-3-((1H-imidazol-5yl)methylene)-2,3-dihydro-1H-indol-5-yl]-2-(6,7-dimethoxy-3,4-dihydroisoquinolin-2-(1H)-yl)acetamide

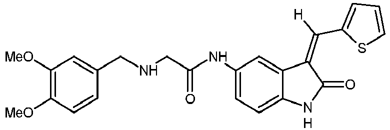

### N-[(3Z)-2-oxo-3-(thiophene-2-ylmethylidene)-2,3-dihydro-1H-indol-5-yl]-2-(3,4-dimethoxybenzylamino)acetamide

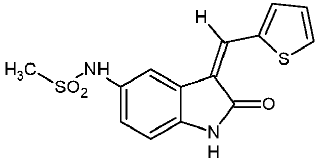

### N-[(3Z)-2-oxo-3-(thiophene-2-ylmethylidene)-2,3-dihydro-1H-indol-5-yl]methanesulfonamide

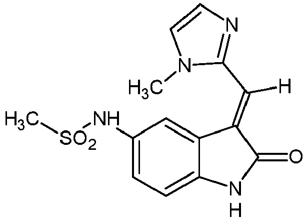

### N-{(3E)-3-[(1-methyl-1H-imidazol-2-yl)methylidene]-2-oxo-2,3-dihydro-1 H-indol-5-yl}methanesulfonamide

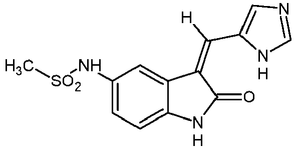

### N-[(3Z)-3-(1H-imidazol-5-ylmethylidene)-2-oxo-2,3-dihydro-1H-indol-5-yl]methanesulfonamide

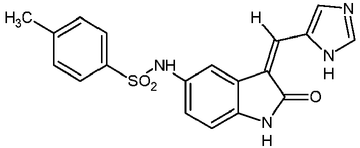

### N-[(3Z)-3-(1H-imidazol-5-ylmethylidene)-2-oxo-2,3-dihydro-1H-indol-5-yl]-4-methylbenzenesulfonamide

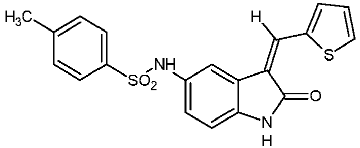

### N-[(3Z)-2-oxo-3-(thiophene-2-ylmethylidene)-2,3-dihydro-1H-indol-5-yl]-4-methylbenzenesulfonamide

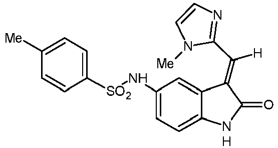

### N-{(3E)-3-[(1-methyl-1H-imidazol-2-yl)methylidene]-2-oxo-2,3-dihydro-1 H-indol-5-yl}-4-methylbenzenesulfonamide.

The at least one 2-oxoindole derivative of the invention can be in the form of a pharmaceutically acceptable salt, preferably selected from the group consisting of hydrochlorides, phosphates, sulfates, oxalates, tartrates, maleates, citrates and succinates.

The compound N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide and the compound N-[(3Z)-2-oxo-3-(thiophene-2-ylmethylidene)-2,3-dihydro-1H-indol-5-yl]-2-(3,4-dimethoxybenzylamino)acetamide are preferably in the form of hydrochlorides.

Preferably the at least one 2-oxoindole derivative of the invention is N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide

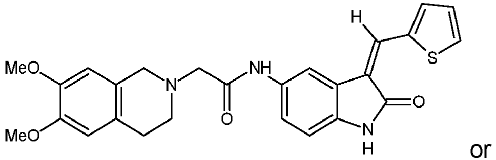

### N-[(3Z)-3-(1H-imidazol-5-ylmethylidene)-2-oxo-2,3-dihydro-1H-indol-5-yl]-4-methylbenzenesulfonamide, more preferably in the form of hydrochloride

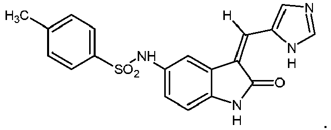

According to the present disclosure the at least one antitumor drug is preferably a chemotherapy or radiotherapy agent, more preferably already known for the treatment of a specific tumor.

According to the present disclosure the at least one antitumor agent is selected from temozolomide, 5-fluorouracil, gemcitabine and temozolomide, cisplatin, paclitaxel and doxorubicin.

The combination of the invention comprises N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide and temozolomide.

In a first preferred and advantageous form, the combination of the invention comprises N-[(3Z)-3-(1H-imidazol-5-ylmethylidene)-2-oxo-2,3-dihydro-1H-indol-5-yl]-4-methylbenzenesulfonamide and temozolomide.

The combination of the invention can be used to treat a tumor.

Therefore the invention concerns a combination comprising at least one 2-oxo-indole derivative of formula (I) 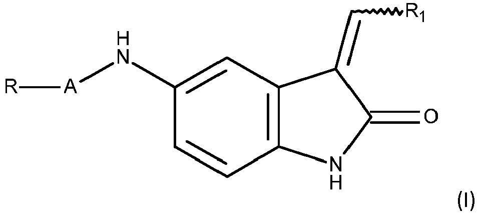 wherein
R₁ is selected from the group consisting of thienyl, imidazole and pyridyl, optionally substituted by (C₁-C₃) alkyl,
A is a -CH₂CO-o-SO₂- group;
R is selected from the group consisting of 6,7-dimethoxy-1,2,3,4-tetrahydroisoquinolinyl, 3,4-dimethoxy-benzylamino, (C₁-C₃)alkyl, benzyl or a pharmaceutically acceptable salt thereof and temozolomide for use in the treatment of tumors.

The tumor for which the combination of the invention is used is preferably glioblastoma multiforme (GBM)), breast tumor and pancreatic tumor. Advantageously and surprisingly the combination of the invention was effective against tumors resistant to the chemotherapy and/or radiotherapy agent.

In the preferred and advantageous form of the invention, the combination is preferably used in the treatment of glioblastoma multiforme (GBM). Advantageously, according to the invention, the combination of the invention has shown synergic effects, in particular constituting a valid pharmacological strategy for the treatment of chemoresistant tumor forms.

### Experimental part

### Example 1: Preparation of the 2-oxoindole derivative compounds

In the following experimental part, all the 2-oxoindole derivative compounds were prepared following the indications provided in "Synthesis of new enzyme inhibitors as potential tools for the antineoplastic therapy"-PhD thesis in Drug Science and Bioactive Substances- XXIV cycle- G. Nesi.

The derivatives 1-5 were synthesized following the synthetic procedure reported in scheme 1 of Figure 1.

The 5-nitro-1,3-dihydro-2*H*-indol-2-one commercial compound was reduced to the corresponding 5-amino-1,3-dihydro-2H-indol-2-one by means of catalytic hydrogenation using Pd/C as a catalyst. The subsequent reaction with the chloro acetyl chloride provided 2-chloro-*N*-(2-oxo-2,3-dihydro-1*H*-indol-5-yl)acetamide which was condensed with the appropriate amine derivative to provide, respectively, 2-[(6,7-dimethoxy-3,4-dihydroisoquinol-2-(1H)-yl)]-N-(2-oxo-2,3-dihydro-1H-indol-5-yl)acetamide and 2-[(3,4-dimethoxybenzyl)amino]-*N*-(2-oxo-2,3-dihydro-1*H*-indol-5-yl)acetamide. Lastly, condensation of the appropriate aromatic carbaldehyde in the presence of pyrrolidine provided the desired products 1-5.

The compounds 6-11 were prepared following the synthetic procedure reported in scheme 2 of Figure 2.

The condensation reaction of 5-amino-1,3-dihydro-2H-indol-2-one with the tosyl chloride or with the mesyl chloride provided, respectively, *N*-(2-oxo-2,3-dihydro-1*H*-indol-5-yl)-4-methylbenzenesulfonamide and *N*-(2-oxo-2,3-dihydro-1*H*-indol-5-yl)methanesulfonamide. The products obtained were subjected to a condensation reaction with the appropriate aromatic carbaldehydes to provide the desired compounds 6-11.

The following 2-oxo-indole derivatives were then prepared:

### Compound 1:

### N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide hydrochloride

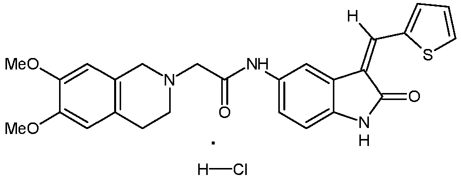

### Compound 2:

### N-[(3E)-(2-oxo-3-((1-methyl-1H-imidazol-2yl)methylene)-2,3-dihydro-1 H-indol-5-yl]-2-(6,7-dimethoxy-3,4-dihydroisoquinol-2-(1 H)-yl)-acetamide

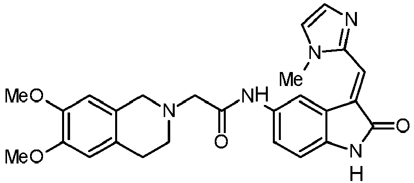

### Compound 3:

### N-[(3E)-(2-oxo-3-((pyridine-2-yl)methylene)-2,3-dihydro-1H-indol-5-yl]-2-(6,7-dimethoxy-3,4-dihydroisoquinolin-2-(1H)-yl)-indolin-5-yl)acetamide

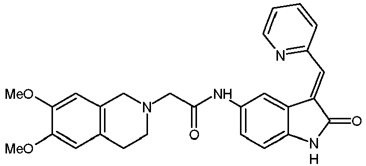

### Compound 4:

### N-[(3Z)-(2-oxo-3-((1H-imidazol-5yl)methylene)-2,3 dihydro-1H-indol-5-yl]-2-(6,7-dimethoxy-3,4-dihydroisoquinolin-2-(1H)-yl)acetamide

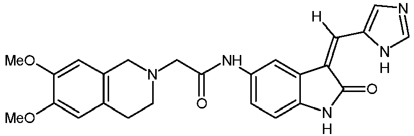

### Compound 5:

### N-[(3Z)-(2-oxo-3-((1H-imidazol-5yl)methylene)-2,3 dihydro-1H-indol-5-yl]-2-(6,7-dimethoxy-3,4-dihydroisoquinolin-2-(1H)-yl)acetamide hydrochloride

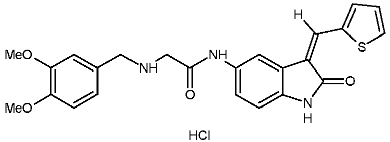

### Compound 6:

### N-[(3Z)-2-oxo-3-(thiophene-2-ylmethylidene)-2,3-dihydro-1H-indol-5-yl]methanesulfonamide

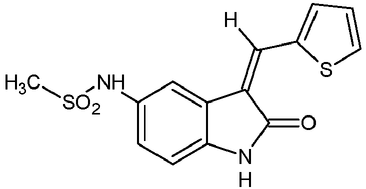

### Compound 7:

### N-{(3E)-3-[(1-methyl-1H-imidazol-2-yl)methylidene]-2-oxo-2,3-dihydro-1 H-indol-5-yl}methanesulfonamide

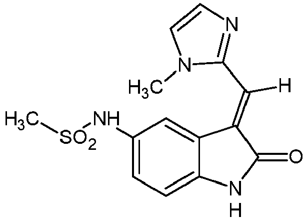

### Compound 8:

### N-[(3Z)-3-(1H-imidazol-5-ylmethylidene)-2-oxo-2,3-dihydro-1H-indol-5-yl]methanesulfonamide

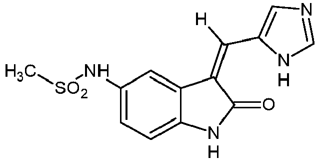

### Compound 9:

### N-[(3Z)-3-(1H-imidazol-5-ylmethylidene)-2-oxo-2,3-dihydro-1H-indol-5-yl]-4-methylbenzenesulfonamide

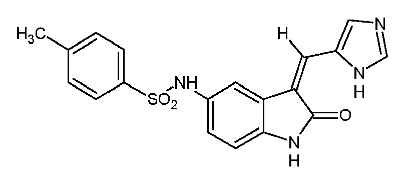

### Compound 10:

### N-[(3Z)-2-oxo-3-(thiophene-2-ylmethylidene)-2,3-dihydro-1H-indol-5-yl]-4-methylbenzenesulfonamide

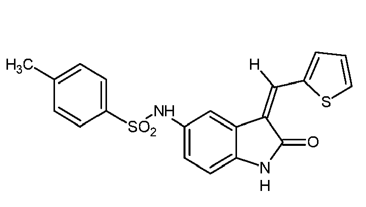

### Compound 11:

### N-{(3E)-3-[(1-methyl-1H-imidazol-2-yl)methylidene]-2-oxo-2,3-dihydro-1 H-indol-5-yl}-4-methylbenzenesulfonamide

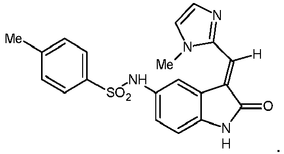

The compounds 1 and 9 were used in combination with temozolomide (TMZ) in the treatment of glioblastoma cell lines.

Temozolomide is a first-line drug for the treatment of glioblastoma; unfortunately, its use in therapy is limited due to the onset of resistance which guarantees survival of the cancer cells (Carmo A, Carvalheiro H, Crespo I, et al. Effect of temozolomide on the U-118 glioma cell line. Oncol Lett 2011;2(6):1165-1170).

### Example 2. Combination comprising compound 9 and temozolomide (TMZ)

Specifically, Glioblastoma Multiforme cells, named U87MG, were used.

The human cells of glioblastoma multiforme U87MG were obtained from the national cancer research institute in Genoa (Italy) and were monitored to determine the DNA profiling. The U87MG cells were cultivated in RPMI medium with the addition of 10% FBS, 2 mM of L-glutamine, 100 U/ml of penicillin, 100 mg/ml of streptomycin and 1% of non-essential amino acids at 37°C in 5% CO₂. The cells were used in the successive experiments up to the fourth detaching passage.

The U87MG cells were grown at 37°C in a humid atmosphere in the presence of 5% CO_{2.} and were placed in a plate at a density of 3,000 cells/well. After 24 hours, the culture medium was replaced with fresh culture medium containing different concentrations of the compound 9 solubilized in DMSO. After 72 hours, the cell viability was assayed by means of MTS assay according to the manufacturer's instructions. Briefly, the assay consists in the reduction of 3-(4,5-dimethylthiazol-2-yl)-5-(3-carboxymethoxyphenyl)-2-(4-sulfophenyl)-2H-tetrazolium (MTS) to soluble formazan by the mitochondrial dehydrogenase. The formazan formed is determined via reading of the absorbance at 490 nM measured with an automated plate reader (Victor Wallac 2, Perkin Elmer). Each experiment was conducted in triplicate. The results were calculated by subtracting the mean background value from the values obtained from each evaluation and were expressed as a control percentage (non-treated cells). To examine the potential synergic effect of cell growth inhibition, the cells of human Glioblastoma Multiforme (GBM) U87MG were treated for 72 hours with increasing concentrations of compound 9 (100 nM, 500 nM, 1 µM), in the absence or in the presence of temozolomide (TMZ, 10 µM or 100 µM). At the end of the treatments, the cell viability was determined by means of MTS assay. The results obtained are shown in Figure 1.

The results obtained showed that compound 9 alone inhibits cell viability, in a concentration-dependent way, with significant effect starting from 500 nM (Figure 1). The compound 9 combined with temozolomide induces a significant increase in inhibition of the cell viability with respect to the single treatment with compound 9 (at all concentrations tested) or with temozolomide (at 10 µM and at 100 µM). Said data show that the combination of compound 9 with temozolomide produces a synergic effect on blocking of the glioblastoma cell proliferation.

### Example 3: Combination comprising compound 1 and temozolomide (TMZ)

The cell line ANGM-CSS and U118-MG were used, human glioblastoma multiforme (GBM) lines. The ANGM-CSS cell line (ECACC cat n°08040401) was supplied by Dr. Angelo Notarangelo, Laboratorio di Citogenetica, IRCCS Casa Sollievo della Sofferenza, San Giovanni Rotondo (FG), Italy. The U118-MG line was purchased from ATCC, Manassas, VA, USA. The culture medium consisted of DMEM:HAMS F12 (1:1) (Sigma-Aldrich) with the addition of 2mM of L-Glutamine (Sigma Aldrich), 10% Fetal Bovine Serum (FBS, Sigma-Aldrich), 100 units/ml penicillin and 100µg/ml streptomycin (P/S) at 37°C in an atmosphere with 5% CO₂.

The cytotoxicity was evaluated via a colorimetric assay, using WST-1 (4-[3-(4-iodophenyl)-2-(4-nitrophenyl)-2H-5-tetrazolium]-1,3-benzene disulfonate) (Roche, Mannheim, Germany). The inhibition of the cell proliferation is expressed as a percentage of the absorbance at 450nm relative to the non-treated cells in culture. The absorbance was measured by an Enspire microplate reader (PerkinElmer, Wellesley, MA, USA).

The compound 1 was tested on the two cell lines of human glioblastoma multiforme ANGM-CSS and U118MG, which are resistant to temozolomide. In particular, as regards the cell line ANGM-CSS, it shows an amplification of the genes MET and EGFR (Notarangelo A, Trombetta D, Parrella P et al. Establishment and genetic characterization of ANGM-CSS, a novel, immortal cell line derived from a human glioblastoma multiforme. Int J Oncol. 2013:10.3892/ijo.2013.2224), whereas the U118MG presents a PTEN mutation (Verreault M, Weppler SA, Stegeman A et al. Combined RNAi-Mediated Suppression of Rictor and EGFR Resulted in Complete Tumor Regression in an Orthotopic Glioblastoma Tumor Model PLoSOne. 2013;8(3):e59597).

The results are shown in Figure 2.

The administration of the compound 1 alone (10µM and 100µM) induces a significant reduction of cell viability in the U118-MG. In this cell line, temozolomide alone (100µM) causes a reduction in viability. The association of the compound 1 with temozolomide induces a synergic inhibitory effect on cell growth.

In the ANGM-CSS, temozolomide alone (100 µM) has no significant effect on cell viability, said result was observed also following administration of compound 1 alone at 10µM. On the other hand, treatment with compound 1 alone at a concentration 10 times higher (100µM) induces a significant reduction in cell viability. The co-administration of compound 1 (10µM) and temozolomide (100µM) causes a significant reduction in viability which is more marked following co-administration of the compound 1 and temozolomide at 100µM.

From the results reported, it is evident that co-administration of the compound 1 together with temozolomide represents a valid pharmacological strategy for the treatment of chemoresistant forms of glioblastoma.

## Claims

1. A combination comprising at least one 2-oxo-indole derivative of formula (I) 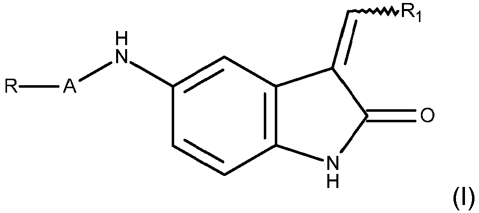 wherein
R₁ is selected from the group consisting of thienyl, imidazolyl and pyridyl, optionally substituted by (C₁-C₃)alkyl,
A is a -CH₂CO- or -SO₂- group;
R is selected from the group consisting of 6,7-dimethoxy-1,2,3,4-tetrahydroisoquinolinyl, 3,4-dimethoxy-benzylamino, (C₁-C₃)alkyl, benzyl or a pharmaceutically acceptable salt thereof and temozolomide.

2. The combination of claim 1, wherein R₁ is thienyl or imidazolyl.

3. The combination of claim 1 or 2, wherein R is selected from the group consisting of 6,7-dimethoxy-1,2,3,4-tetrahydroisoquinolinyl, 3,4-dimethoxy-benzylamino, methyl and benzyl.

4. The combination of claim 3, wherein R is 6,7-dimethoxy-1,2,3,4-tetrahydroquinolinyl or benzyl.

5. The combination according to claim 1, wherein the at least one 2-oxoindole derivative is a compound selected from the group consisting of 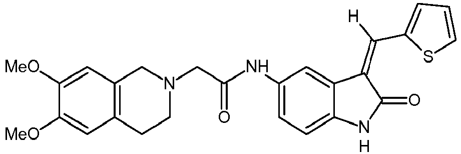 N-[(3Z)-(2-oxo-3-(thiophene-2-ylmethylene)-2,3-dihydro-1H-indol-5-yl]-2-(6,7-dimethoxy-3,4-dihydroisoquinol-2-(1H)-yl)-acetamide 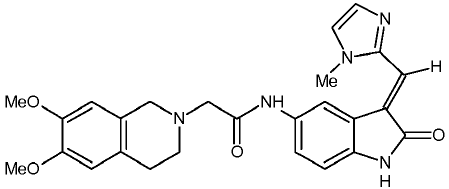 N-[(3E)-(2-oxo-3-((1-methyl-1H-imidazol-2yl)methylene)-2,3-dihydro-1 H-indol-5-yl]-2-(6,7-dimethoxy-3,4-dihydroisoquinol-2-(1H)-yl)-acetamide 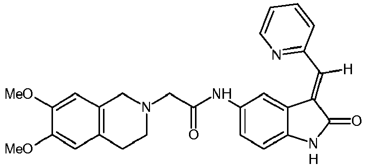 N-[(3E)-(2-oxo-3-((pyridine-2-yl)methylene)-2,3-dihydro-1H-indol-5-yl]-2-(6,7-dimethoxy-3,4-dihydroisoquinolin-2-(1H)-yl)-indolin-5-yl)acetamide 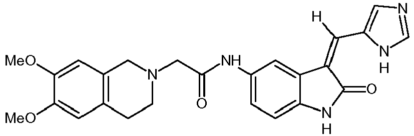 N-[(3Z)-(2-oxo-3-((1H-imidazol-5yl)methylene)-2,3 dihydro-1H-indol-5-yl]-2-(6,7-dimethoxy-3,4-dihydroisoquinolin-2-(1H)-yl)acetamide 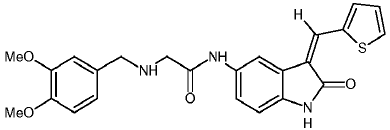 N-[(3Z)-2-oxo-3-(thiophen-2-ylmethylidene)-2,3-dihydro-1H-indol-5-yl]-2-(3,4-dimethoxybenzylamino)acetamide 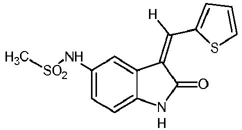 N-[(3Z)-2-oxo-3-(thiophen-2-ylmethylidene)-2,3-dihydro-1H-indol-5-yl]methanesulfonamide 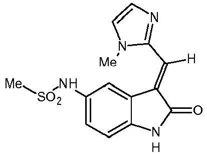 N-{(3E)-3-[(1-methyl-1H-imidazol-2-yl)methylidene]-2-oxo-2,3-dihydro-1 H-indol-5-yl}methanesulfonamide 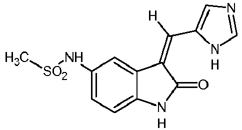 N-[(3Z)-3-(1H-imidazol-5-ylmethylidene)-2-oxo-2,3-dihydro-1H-indol-5-yl]methanesulfonamide 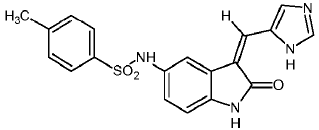 N-[(3Z)-3-(1H-imidazol-5-ylmethylidene)-2-oxo-2,3-dihydro-1H-indol-5-yl]-4-methylbenzenesulfonamide 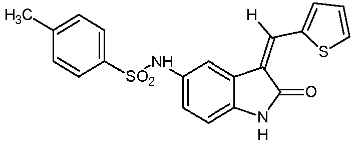 N-[(3Z)-2-oxo-3-(thiophen-2-ylmethylidene)-2,3-dihydro-1H-indol-5-yl]-4-methylbenzenesulfonamide 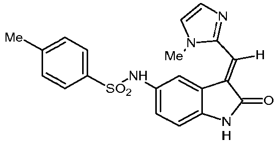 N-{(3E)-3-[(1-methyl-1H-imidazol-2-il)methylidene]-2-oxo-2,3-dihydro-1 H-indol-5-yil}-4-methylbenzenesulfonamide.

6. The combination of claim 5, wherein the at least one 2-oxoindole derivative is N-[(3Z)-(2-oxo-3-(thiophen-2-ylmethylene)-2,3-dihydro-1H-indol-5-yl]-2-(6,7-dimethoxy-3,4-dihydroisoquinol-2-(1H)-yl)-acetamide or N-[(3Z)-3-(1H-imidazol-5-ylmethylidene)-2-oxo-2,3-dihydro-1H-indol-5-yl]-4-methyl benzenesulfonamide.

7. The combination according to any one of claims 1 to 6, wherein said pharmaceutically acceptable salt is selected from the group consisting of hydrochloride, phosphate, sulfate, oxalate, tartrate, maleate, citrate and succinate.

8. The combination according to claim 1 comprising N-[(3Z)-(2-oxo-3-(thiophen-2-ylmethylene)-2,3-dihydro-1H-indol-5-yl]-2-(6,7-dimethoxy-3,4-dihydroisoquinol-2-(1H)-yl)-acetamide and temozolomide.

9. The combination according to claim 1 comprising N-[(3Z)-3-(1H-imidazol-5-ylmethylidene)-2-oxo-2,3-dihydro-1H-indol-5-yl]-4-methylbenzenesulfonamide and temozolomide.

10. A combination comprising at least one 2-oxo-indole derivative of formula (I) 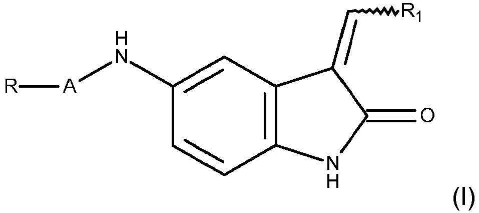 wherein
R₁ is selected from the group consisting of thienyl, imidazolyl and pyridyl, optionally substituted by (C₁-C₃) alkyl,
A is a-CH₂CO- or -SO₂- group;
R is selected from the group consisting of 6,7-dimethoxy-1,2,3,4-tetrahydroisoquinolinyl, 3,4-dimethoxy-benzylamino, (C₁-C₃)alkyl, benzyl or a pharmaceutically acceptable salt thereof and temozolomide for use in the treatment of tumors.

11. The combination for use according to claim 10, wherein the tumor is glioblastoma multiforme (GBM), breast tumor, pancreatic tumor.

12. The combination for use according to claim 11, wherein the tumor is glioblastoma multiforme (GBM).

13. The combination for use according to claim 9, wherein the tumor is a tumor resistant to the chemotherapeutic and/or radiotherapeutic agent.

14. The combination for use according to claim 13, wherein the tumor is glioblastoma.

## Patentansprüche

1. Verbindung, zumindest ein 2-oxo-indol-Derivat der Formel (I) 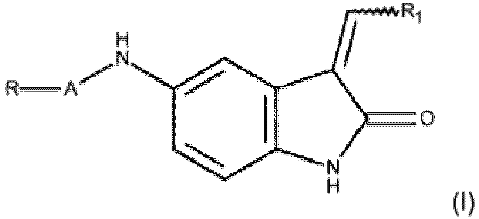 aufweisend, wobei
- R₁ ausgewählt ist aus der Gruppe bestehend aus Thienyl, Imidazolyl und Pyridyl, wahlweise substituiert durch (C₁-C₃)alkyl,
- A eine -CH₂CO- oder -SO₂- Gruppe ist;
- R ausgewählt ist aus der Gruppe bestehend aus 6,7-dimethoxy-1,2,3,4-tetrahydroisoquinolinyl, 3,4-dimethoxy-benzylamino, (C₁-C₃)alkyl, Benzyl oder einem pharmazeutisch zulässige Salz davon und Temozolomid.

2. Verbindung gemäß Anspruch 1, wobei R₁ Thienyl oder Imidazolyl ist.

3. Verbindung gemäß Anspruch 1 oder 2, wobei R ausgewählt ist aus der Gruppe bestehend aus 6,7-dimethoxy-1,2,3,4-tetrahydroisoquinolinyl, 3,4-dimethoxy-benzylamino, Methyl und Benzyl.

4. Verbindung gemäß Anspruch 3, wobei R 6,7-dimethoxy-1,2,3,4-tetrahydroisoquinolinyl oder Benzyl ist.

5. Verbindung gemäß Anspruch 1, wobei das zumindest eine 2-oxoindol-Derivat eine Zusammensetzung ist, die ausgewählt ist aus der Gruppe bestehend aus 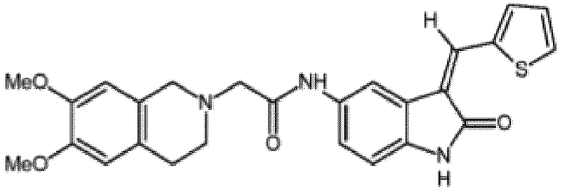 N-[(3Z)-(2-oxo-3-(thiophen-2-ylmethylen)-2,3-dihydro-1H-indol-5-yl]-2-(6,7-dimethoxy-3,4-dihydroisoquinol-2-(1H)-yl)-acetamid 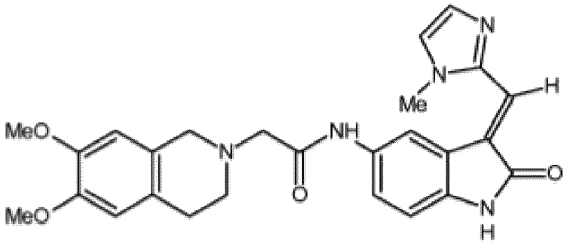 N-[(3E)-(2-oxo-3-((1-methyl-1H-imidazol-2yl)methylen)-2,3-dihydro-1H-indol-5-yl]-2-(6,7-dimethoxy-3,4-dihydroisoquinol-2-(1H)-yl)-acetamid 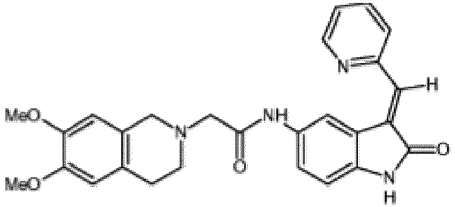 N-[(3E)-(2-oxo-3-((pyridin-2-yl)methylen)-2,3-dihydro-1H-indol-5-yl]-2-(6,7-dimethoxy-3,4-dihydroisoquinolin-2-(1H)-yl)-indolin-5-yl)acetamid 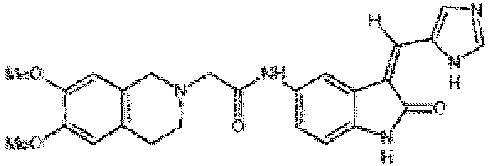 N-[(3Z)-(2-oxo-3-((1H-imidazol-5yl)methylen)-2,3 dihydro-1H-indol-5-yl]-2-(6,7-dimethoxy-3,4-dihydroisoquinolin-2-(1H)-yl)acetamid 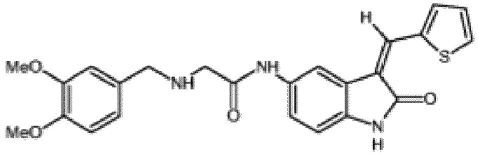 N-[(3Z)-2-oxo-3-(thiophen-2-ylmethyliden)-2,3-dihydro-1H-indol-5-yl]-2-(3,4-dimethoxybenzylamino)acetamid 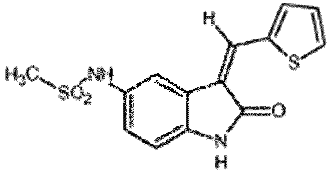 N-[(3Z)-2-oxo-3-(thiophen-2-ylmethyliden)-2,3-dihydro-1H-indol-5-yl]methansulfonamid 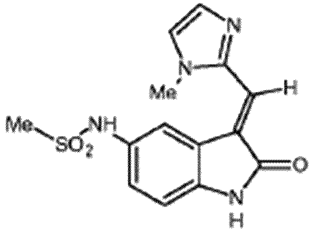 N-{(3E)-3-[(1-methyl-1H-imidazol-2-yl)methyliden]-2-oxo-2,3-dihydro-1H-indol-5-yl}methansulfonamid 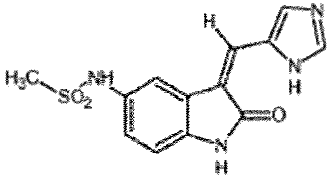 N-[(3Z)-3-(1H-imidazol-5-ylmethyliden)-2-oxo-2,3-dihydro-1H-indol-5-yl]methansulfonamid 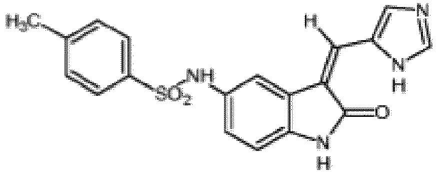 N-[(3Z)-3-(1H-imidazol-5-ylmethyliden)-2-oxo-2,3-dihydro-1H-indol-5-yl]-4-methylbenzensulfonamid 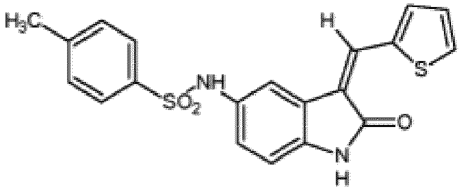 N-[(3Z)-2-oxo-3-(thiophen-2-ylmethyliden)-2,3-dihydro-1H-indol-5-yl]-4-methylbenzensulfonamid 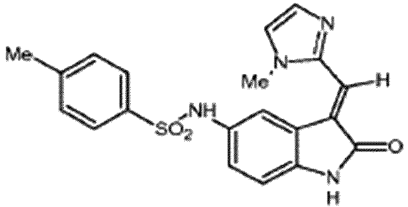 N-{(3E)-3-[(1-methyl-1H-imidazol-2-il)methyliden]-2-oxo-2,3-dihydro-1H-indol-5-yil}-4-methylbenzensulfonamid.

6. Verbindung gemäß Anspruch 5, wobei das zumindest eine 2-oxoindol-Derivat N-[(3Z)-(2-oxo-3-(thiophen-2-ylmethylen)-2,3-dihydro-1H-indol-5-yl]-2-(6,7-dimethoxy-3,4-dihydroisoquinol-2-(1H)-yl)-acetamid
oder
N-[(3Z)-3-(1H-imidazol-5-ylmethyliden)-2-oxo-2,3-dihydro-1H-indol-5-yl]-4-methylbenzensulfonamid
ist.

7. Verbindung gemäß einem der Ansprüche 1 bis 6, wobei das pharmazeutisch zulässige Salz ausgewählt ist aus der Gruppe bestehend aus Hydrochlorid, Phosphat, Sulfat, Oxalat, Tartrat, Maleat, Zitrat und Succinat.

8. Verbindung gemäß Anspruch 1, aufweisend N-[(3Z)-(2-oxo-3-(thiophen-2-ylmethylen)-2,3-dihydro-1H-indol-5-yl]-2-(6,7-dimethoxy-3,4-dihydroisoquinol-2-(1H)-yl)-acetamid und Temozolomid.

9. Verbindung gemäß Anspruch 1, aufweisend N-[(3Z)-3-(1H-imidazol-5-ylmethyliden)-2-oxo-2,3-dihydro-1H-indol-5-yl]-4-methylbenzensulfonamid und Temozolomid.

10. Verbindung, aufweisend zumindest ein 2-oxo-indol-Derivat der Formel (I) 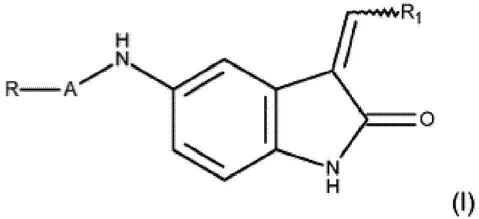 wobei
R₁ ausgewählt ist aus der Gruppe bestehend aus Thienyl, Imidazolyl und Pyridyl, wahlweise substituiert durch (C₁-C₃)alkyl,
A eine -CH₂CO- oder -SO₂- Gruppe ist;
R ausgewählt ist aus der Gruppe bestehend aus 6,7-dimethoxy-1,2,3,4-tetrahydroisoquinolinyl, 3,4-dimethoxy-benzylamino, (C₁-C₃)alkyl, Benzyl oder einem pharmazeutisch zulässigen Salz davon und Temozolomid zur Verwendung bei der Behandlung von Tumoren.

11. Verbindung zur Verwendung gemäß Anspruch 10, wobei der Tumor Glioblastoma multiforme (GBM), Brustkrebs, Pankreastumor ist.

12. Verbindung zur Verwendung gemäß Anspruch 11, wobei der Tumor Glioblastoma multiforme (GBM) ist.

13. Verbindung zur Verwendung gemäß Anspruch 9, wobei der Tumor ein Tumor ist, der gegenüber chemotherapeutischen und / oder radiotherapeutischen Wirkstoffen resistent ist.

14. Verbindung zur Verwendung gemäß Anspruch 13, wobei der Tumor Glioblastoma ist.

## Revendications

1. Combinaison comprenant au moins un dérivé de 2-oxo-indole de formule (I) 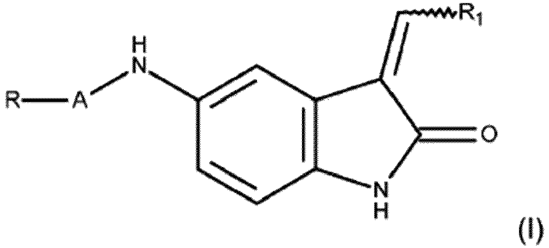 dans laquelle
R₁ est sélectionné parmi le groupe constitué de thiényle, imidazolyle et pyridyle, facultativement substitué par (C₁-C₃)alkyle,
A est un groupe -CH₂CO- ou -SO₂- ;
R est sélectionné parmi le groupe constitué de 6,7-diméthoxy-1,2,3,4-tétrahydroisoquinolinyle, 3,4-diméthoxy-benzylamino, (C₁-C₃)alkyle, benzyle ou un sel pharmaceutiquement acceptable de ceux-ci et témozolomide.

2. Combinaison de la revendication 1, dans laquelle R₁ est thiényle ou imidazolyle.

3. Combinaison de la revendication 1 ou 2, dans laquelle R est sélectionné parmi le groupe constitué de 6,7-diméthoxy-1,2,3,4-tétrahydroisoquinolinyle, 3,4-diméthoxy-benzylamino, méthyle et benzyle.

4. Combinaison de la revendication 3, dans laquelle R est 6,7-diméthoxy-1,2,3,4-tétrahydroquinolinyle ou benzyle.

5. Combinaison selon la revendication 1, dans laquelle l'au moins un dérivé de 2-oxoindole est un composé sélectionné parmi le groupe constitué de 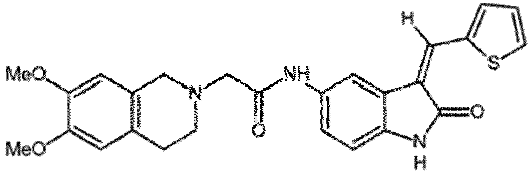 N-[(3Z)-(2-oxo-3-(thiophène-2-ylméthylène)-2,3-dihydro-1H-indol-5-yl]-2-(6,7-diméthoxy-3,4-dihydroisoquinol-2-(1H)-yl)-acétamide 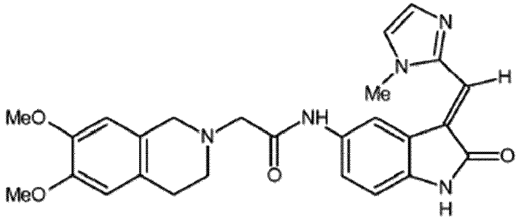 N-[(3E)-(2-oxo-3-((1-méthyl-1H-imidazol-2yl)méthylène)-2,3-dihydro-1H-indol-5-yl]-2-(6,7-diméthoxy-3,4-dihydroisoquinol-2-(1H)-yl)-acétamide 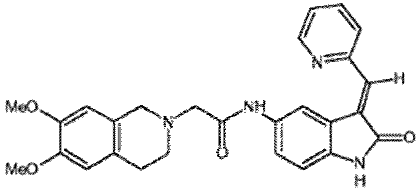 N-[(3E)-(2-oxo-3-((pyridine-2-yl)méthylène)-2,3-dihydro-1H-indol-5-yl]-2-(6,7-diméthoxy-3,4-dihydroisoquinolin-2-(1H)-yl)-indolin-5-yl)acétamide 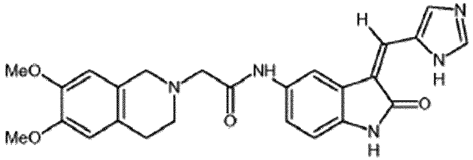 N-[(3Z)-(2-oxo-3-((1H-imidazol-5yl)méthylène)-2,3 dihydro-1H-indol-5-yl]-2-(6,7-diméthoxy-3,4-dihydroisoquinolin-2-(1H)-yl)acétamide 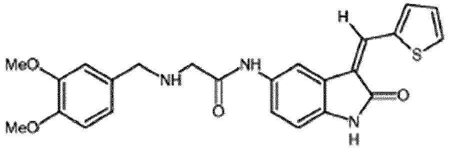 N-[(3Z)-2-oxo-3-(thiophén-2-ylméthylidène)-2,3-dihydro-1 H-indol-5-yl]-2-(3,4-diméthoxybenzylamino)acétamide 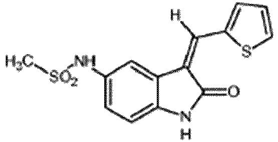 N-[(3Z)-2-oxo-3-(thiophén-2-ylméthylidène)-2,3-dihydro-1 H-indol-5-yl]méthanesulfonamide 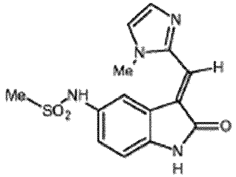 N-{(3E)-3-[(1-méthyl-1H-imidazol-2-yl)méthylidène]-2-oxo-2,3-dihydro-1 H-indol-5-yl}méthanesulfonamide 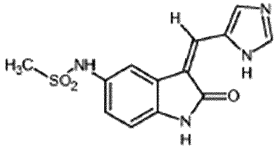 N-[(3Z)-3-(1H-imidazol-5-ylméthylidène)-2-oxo-2,3-dihydro-1H-indol-5-yl]méthanesulfonamide 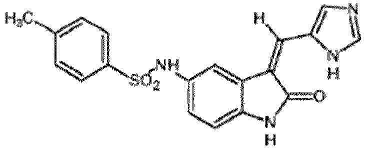 N-[(3Z)-3-(1H-imidazol-5-ylméthylidène)-2-oxo-2,3-dihydro-1H-indol-5-yl]-4-méthylbenzènesulfonamide 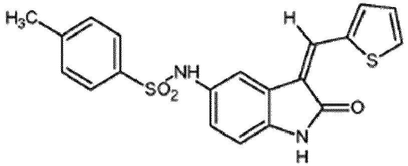 N-[(3Z)-2-oxo-3-(thiophén-2-ylméthylidène)-2,3-dihydro-1 H-indol-5-yl]-4-méthylbenzènesulfonamide 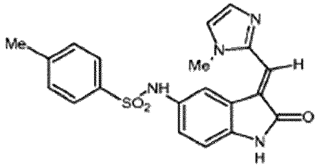 N-{(3E)-3-[(1-méthyl-1H-imidazol-2-yl)méthylidène]-2-oxo-2,3-dihydro-1 H-indol-5-yl}-4-méthylbenzènesulfonamide.

6. Combinaison de la revendication 5, dans laquelle l'au moins un dérivé de 2-oxoindole est N-[(3Z)-(2-oxo-3-(thiophén-2-ylméthylène)-2,3-dihydro-1H-indol-5-yl]-2-(6,7-diméthoxy-3,4-dihydroisoquinol-2-(1H)-yl)-acétamide ou N-[(3Z)-3-(1H-imidazol-5-ylméthylidène)-2-oxo-2,3-dihydro-1H-indol-5-yl]-4-méthylbenzènesulfonamide.

7. Combinaison selon l'une quelconque des revendications 1 à 6, dans laquelle ledit sel pharmaceutiquement acceptable est sélectionné parmi le groupe constitué de chlorhydrate, phosphate, sulfate, oxalate, tartrate, maléate, citrate et succinate.

8. Combinaison selon la revendication 1, comprenant N-[(3Z)-(2-oxo-3-(thiophén-2-ylméthylène)-2,3-dihydro-1H-indol-5-yl]-2-(6,7-diméthoxy-3,4-dihydroisoquinol-2-(1 H)-yl)-acétamide et témozolomide.

9. Combinaison selon la revendication 1, comprenant N-[(3Z)-3-(1H-imidazol-5-ylméthylidène)-2-oxo-2,3-dihydro-1H-indol-5-yl]-4-méthylbenzènesulfonamide et témozolomide.

10. Combinaison comprenant au moins un dérivé de 2-oxo-indole de formule (I) 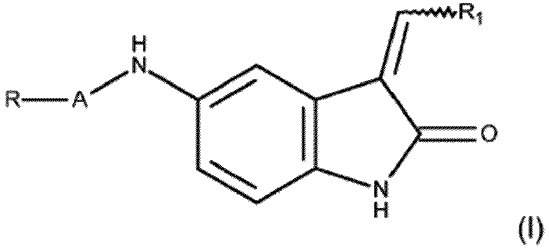 dans laquelle
R₁ est sélectionné parmi le groupe constitué de thiényle, imidazolyle et pyridyle, facultativement substitué par (C₁-C₃) alkyle,
A est un groupe -CH₂CO- ou -SO₂- ;
R est sélectionné parmi le groupe constitué de 6,7-diméthoxy-1,2,3,4-tétrahydroisoquinolinyle, 3,4-diméthoxy-benzylamino, (C₁-C₃)alkyle, benzyle ou un sel pharmaceutiquement acceptable de ceux-ci et témozolomide pour une utilisation dans le traitement de tumeurs.

11. Combinaison pour une utilisation selon la revendication 10, dans laquelle la tumeur est un glioblastome multiforme (GBM), une tumeur du sein, une tumeur du pancréas.

12. Combinaison pour une utilisation selon la revendication 11, dans laquelle la tumeur est un glioblastome multiforme (GBM).

13. Combinaison pour une utilisation selon la revendication 9, dans laquelle la tumeur est une tumeur résistante à l'agent chimiothérapeutique et/ou radiothérapeutique.

14. Combinaison pour une utilisation selon la revendication 13, dans laquelle la tumeur est un glioblastome.
